# EUROPEAN PATENT APPLICATION

(11) **EP 1 726 962 A1**
(43) Date of publication of application: **29.11.2006**
(21) Application number: 05104421.2
(22) Date of filing: 24.05.2005
(51) Int. Cl.: G01N 33/68

(54) **Apolipoprotein E plasma levels for monitoring and reducing the risk of cardiovascular disease**

(71) Applicant: Leiden University Medical Center, 2333 ZA Leiden (NL)
(72) Inventor: Mooijaart, Simon Pieter, 2331 SH, Leiden (NL); Berbee, Jimmy Fransiscus Paulus, 2317 CG, Leiden (NL); Van Heemst, Everdina Wilhelmina, 2716 HV, Zoetermeer (NL); Havekes, Aloysius Maria, 2402 PP, Alphen aan den Rijn (NL); De Craen, Antonius Jacobus Maria, 2313 SV, Leiden (NL); Slagboom, Pieternella, 2481 BB, Woubrugge (NL); Rensen, Patrick Cornelis Nicolaas, 2317 MG, Leiden (NL); Westendorp, Rudolf Gerardus Johannes, 2317 KE, Leiden (NL)
(74) Representative: van Westenbrugge, Andries

(57) **Abstract**

The current invention exploits the presence of a newly established functional correlation between plasma levels of apolipoprotein E (apoE) and cardiovascular disease (CVD) risk and/or CVD related mortality. The association of apoE plasma levels closely correlates with cardiovascular mortality, independent of *APOE* ε2/ε3/ε4 genotype and even when the model is adjusted for other CVD risk factors. The invention is first aimed at diagnostic methods for the determination of the risk of a subject for developing CVD and mortality related to CVD. The invention is also aimed at therapeutic intervention of high plasma levels of apoE. The invention achieves prevention of CVD risk by reducing the plasma levels of apoE, for which several embodiments, pharmaceutical and nutraceutical compositions with one or more active compounds, are disclosed.

## Description

### Field of the invention

The current invention relates to the field of medicine, in particular the field of lipid-and cholesterol metabolism and diagnostics and treatment of cardiovascular disease.

### Background of the invention

Lipid metabolism and serum levels of triglycerides and fractionated cholesterol, in particular high levels of Low Density Lipoproteins (LDL) cholesterol, low levels of High Density Lipoproteins (HDL) cholesterol or an unfavorably low ratio of HDL/LDL cholesterol, are important risk factors implicated in atherosclerosis and cardiovascular disease (CVD) in general. Unlike the risk of high LDL cholesterol that diminishes in old age, low HDL-cholesterol determines the risk of cardiovascular disease up to the highest age categories (1-4).

Determination of serum levels of cholesterol, in particular the determination of the distribution of cholesterol in 'good' high density lipoprotein complexes and 'bad' low density lipoprotein complexes is rather laborious and prone to analytical variation during the fractionation procedures, usually done by sequential flotation. Moreover, the analytical accuracy obtained with routinely used fractionation procedures is rather limited as these coarsely separate high density lipoprotein complexes from low density lipoprotein complexes. HDL, the major component of the high density fraction, consists of different lipoprotein complexes (such as slow and fast HDL). Although LDL is the main component of the low density fraction, this fraction also contains other low density lipoprotein components, such as IDL (intermediate density), VLDL (very low density) and chylomicrons and their remnants. Most routinely used fractionation procedures thus do not yield detailed information on the relative contribution of all these different lipoprotein complexes in the high or low density fractions. Although more detailed fractionation can be achieved with other techniques, such as chromatography, these are time-consuming and expensive for routine use.

The before mentioned lipoprotein complexes in blood plasma consist of a number of lipids and proteins. Among these proteins are so called apolipoproteins, some of which have been implicated in CVD risk and may be used as markers for cholesterol (sub-)fractions. High plasma levels of Lipoprotein(a) [Lp(a) or apoa] have been shown to be a strong and important risk factor for cardiovascular disease (CVD). In particular the small-sized apoa isoforms (F, B, S1, and S2) are inversely correlated with the high levels of apoa in plasma and significantly associated with CVD. The *Apoa* gene comprises at least 19 different alleles in total, which complicates the use of apoa for determining the risk of CVD.

Serum levels of apolipoprotein B (apo B) are also a factor for evaluating CVD (US 2003/0096316). Apolipoprotein B-100 is the major protein constituent of LDL cholesterol and can be used as a marker for CVD risk.

The apolipoprotein E (*APOE*) gene is another key regulator of lipid metabolism. The apoE protein is a surface component of HDL, triglyceride-rich chylomicrons, very low density lipoprotein (VLDL) and their remnants, and apoE determines the receptor-mediated (the apoB-100/apoE receptor) uptake of these lipoproteins by the liver, thus playing a role in regulation of the circulating levels of cholesterol and triglycerides (1).

The biological activity of apoE can be influenced by modification of it's structure and/or it's relative quantity in circulation. A structural alteration arises from the common ε2/ε3/ε4 polymorphism (5), that affects lipid metabolism (6), and risk of cardiovascular disease (7) and dementia (8). As a consequence of the structural differences between the ε2/ε3/ε4 isoforms of apoE, these isoforms circulate in different concentrations (9,10). The ε2 allele is associated with higher apoE levels and the ε4 allele is associated with lower levels. The structural modification in the apoE2 protein reduces its affinity for the apoB-100/apoE receptor, resulting in higher circulating apoE levels. However, despite the observed association between *APOE* genotype and circulating levels of plasma ApoE, the *APOE* ε2/ε3/ε4 genotype is only a minor determinant of circulating apoE levels. The effect of plasma levels of apoE, independent of *APOE* ε2/ε3/ε4 genotype, on risk of developing CVD have hitherto not been reported.

### Summary of the invention

The current invention exploits the presence of a newly established functional correlation between plasma apoE levels and cardiovascular disease risk and/or CVD related mortality. Unlike the correlation between the apo ε2/ε3/ε4 variants and plasma apoE levels, a correlation between apoE levels, i.e. within a population of subjects having the same apoE variant, has not yet been reported as being an independent risk factor for CVD. It was found by the inventors that the association of apoE plasma levels closely correlates with cardiovascular mortality, independent of *APOE* ε2/ε3/ε4 genotype. Moreover, the correlation is still apparent when the model is adjusted for other CVD risk factors, such as the plasma levels of lipids (triglycerides, LDL and HDL cholesterol), and plasma levels of C reactive protein (CRP, which is a marker of chronic/systemic inflammation) and even when risk-adjusted for the combined factors lipids, CRP and medical history of the subject, such as smoking, hypertension and diabetes. The current invention is aimed at the practical exploitation of the striking and unexpected finding that the correlation of plasma apoE protein levels and CVD risk and/or mortality is independent of other risk factors for CVD. The invention is first aimed at diagnostic methods for the determination of the risk of a subject for developing cardiovascular disease and mortality related to cardiovascular disease. The invention is also aimed at therapeutic intervention of high plasma levels of apoE. The invention achieves prevention of CVD risk by reducing the plasma levels of apoE, for which several embodiments, pharmaceutical and nutraceutical compositions with one or more active compounds, will be disclosed.

### Detailed description of the invention

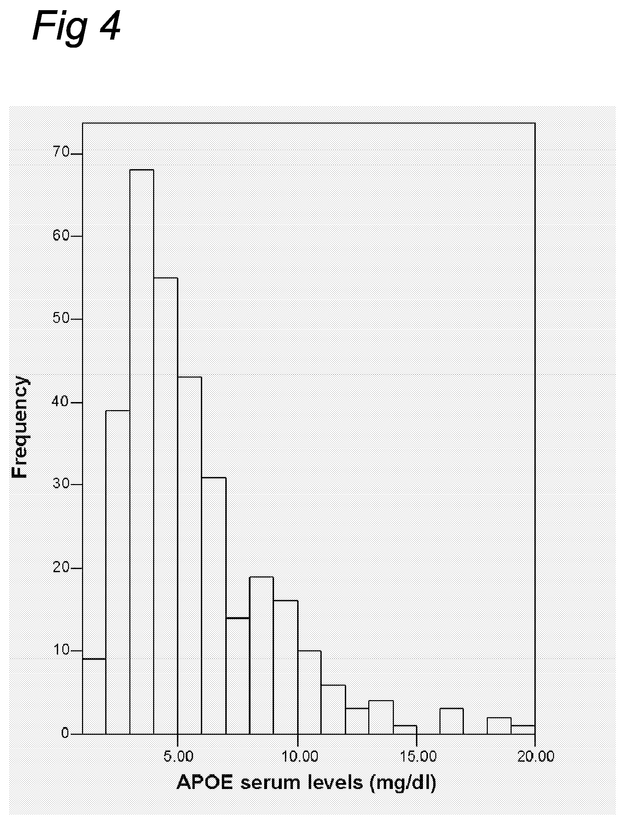

In a first embodiment the invention provides a diagnostic method for determining the risk of a subject for cardiovascular disease, comprising the step of determining the level of Apolipoprotein E (apoE) protein in a bodily fluid. The level of apoE can be an absolute level of apoE, normally measured in milligrams per deciliter of blood plasma, typically varying between <1 up to >10 mg/dL (Figure 4). Preferably, the level of apoE protein in the plasma is correlated to a reference or standard level, which may be an absolute reference level, or which may be a relative reference level, preferably the average of a matched control group of subjects. The matched control group of subjects may be matched for one or more relevant factors, of which the genotype of apolipoprotein E (*APOE*) gene (apo ε2/ε3/ε4 variants) is one of the most relevant. The reference subject or group of subjects may therefore be matched with respect to apoE genotype, for at least one and preferably for both alleles. The control group of the subject can further or preferably additionally, be matched with respect to one or more other factors. These factors may be selected from gender, age, diet and dietary habits (such as, but not limited to daily fat or daily calorie intake), health status and/or history (such as, but not limited to: body mass index (BMI), diabetes, smoking, hypertension, inflammation markers such as CRP (C-reactive protein)), social/cultural/genetic backgrounds, geographical location, plasma lipid levels (such as triglycerides, homocysteine, LDL and HDL cholesterol plasma levels), and other known or to be identified risk factors or potential risk factors for CVD. In a preferred embodiment, when the number of subjects in a population is sufficiently high, stratification of the group may be employed. Subjects and/or observations may be combined into strata (tertiles, quartiles, deciles etc.) with a certain bandwith of apoE plasma levels, such as in the examples 1 and 2 in this specification, wherein the group was divided in tertiles; subpopulations with high, intermediate and low apoE plasma levels, or in deciles. Each subpopulation can subsequently be correlated with a certain risk of developing CVD or CVD mortality, in comparison to the other strata or subgroups.

The diagnostic method of determining apoE plasma levels may be used as a single diagnostic factor or may be used in combination with the determination of other risk factors for developing CVD such as blood pressure, CRP levels, cholesterol levels. In this specification, cardiovascular disease includes all clinical outcomes of the atherosclerotic process as these are described in ICD-10 codes I00-I99, in particular atherosclerosis of the heart, brain and peripheral vascular system (ref: International Statistical Classification of Diseases and Related Health Problems, 10th Revision (ICD-10). Vol 1. Geneva, Switzerland: World Health Organization; 1992. CVD comprises conditions such as but not limited to: congestive heart failure, angina pectoris, myocardial infarction, stroke, claudicatio intermittens, aneurysmata of the large arterial vessels and other (cardio-)vascular diseases.

The level of apoE is determined in or on a bodily fluid, wherein the bodily fluid may be whole blood, blood serum, blood plasma or any derivative or preparation thereof. The blood or blood fractions may optionally be treated for storage with anti-clotting agents known in the art, diluted or otherwise treated for storage, preservation or handling. The blood sample to be analyzed for apoE plasma level may be obtained via routine methods from any mammal, preferably from a human subject. Samples may be taken once, at an arbitrarily chosen point in time, or may be taken at regular intervals, daily, weekly or monthly. Measurements on multiple time points may for instance be advantageous to monitor the effects of dietary or pharmaceutical interventions, diets and/or therapies to decrease the plasma levels of apoE and/or reduce the risk of CVD. Preferably a sample is taken on a fixed time point during the day, for instance after waking up and before having breakfast.

ApoE levels in the blood sample or blood-fraction obtained from a subject may be determined via routine methods known in the art. Preferably, immunodiagnostic methods may be used, although other means such as advanced mass spectrometry techniques may be applied. Antibodies to be used may be raised by the skilled artisan or may be commercially available monoclonal or polyclonal antibodies or fragments (Fab₂, Fab, Fv or scFv) or derivatives thereof, capable of specifically recognizing or binding to apoE protein. Examples of suitable immunodiagnostic methods are enzyme linked immunosorbent assay (ELISA), radio-immuno assays (RIA), western blotting techniques to detect apoE protein separated on gel and transferred on a solid carrier such as a membrane, or protein-, peptide- or antibody-(micro)arrays on membranes, columns, particles, glass- or metal slides or other supports. Both the apoE antigen or the antibody may be labeled directly and/or may immobilized on a carrier before being brought into contact with each other. Detection may take place with dyes, fluorochromes (such as but not limited to FITC, TRITC, Rhodamine, GFP or derivates thereof), enzymatic reactions (such as but not limited to alkaline phosphatase, peroxidase), metal particles (immuno-gold), magnetic particles, radioactive isotopes (such as: ³²P, ³³P, ³⁵S, ¹²⁵I, ³H), immunogenic moieties or molecular tags (such as but not limited to: biotin, digoxigenin, HA or lexA) and other equivalents known in the art and for instance described in Current Protocols in Immunology, Wiley Interscience, 2004, or in Harlow and Lane, Antibodies, a Laboratory Manual, CSH press, 1998. The apoE specific antibody may be labeled directly with the labels mentioned before, or a sandwich technique with use of a secondary antibody or protein (protein A or G, or streptavidin) may be applied. Alternatively, components of the sample, including ApoE, may first be labeled, followed by binding of a specific antibody and visualization.

In another embodiment, the current invention provides pharmaceutical and nutraceutical compositions for the reduction of plasma levels of apoE in a subject. Pharmaceutical compositions according to the invention comprise at least one or more of the compounds selected from the group of compounds capable of reducing apoE plasma levels in a subject, consisting of TNF-α, INF-γ, Lactosyl-Ceramide (Lac-Cer), Glucosyl-Ceramide (Glc-Cer) (refs. 24, 25, 26), phosphonate-phosphate, diphosphonate, aminodiphosphonates, hydroxyphosphonates and phosphonophosphates (WO 03/070169 A2, WO 03/079179 A2, WO 03/094852 A2), HMG-CoA reductase inhibitors (the 'statin' family comprising members such as, but not limited to: mevastatin, lovastatin, compactin, nisvastatin, atorvastatin, pravastatin, simvastatin, fluvastatin), NF-κB inhibitors and isoprenylation inhibitors (US 6,428,950 B1). Compounds reducing apoE plasma levels may act through several mechanisms, comprising but not limited to, inhibition of production of apoE-rich lipoprotein particles or stimulation of the clearance of apoE-rich lipoprotein particles.

In yet another embodiment, the invention pertains to a method for decreasing the risk of a subject for cardiovascular disease, and in particular decreasing the progression of and/or stimulating regression of the process of atherosclerosis and related pathologies, comprising the step of reducing the plasma levels of apoE protein. This aim is achieved by administering to the subject a composition according to the invention in an amount sufficient to reduce plasma levels of apoE protein. The composition according to the invention is preferably administered to a subject at risk of or suffering from cardiovascular disease. Such a subject may have apoE levels that are above the average level in comparison to a matched control or reference group. The reference or control group may be matched, as explained above, with respect to *APOE* genotype, gender, age, health status, plasma lipid levels, diet and/or various other relevant factors. Preferably the composition according to the invention is administered in an amount sufficient to reduce apoE plasma levels with at least 10, 20, 30, 50 or 80% with respect to apoE plasma levels before treatment. Most preferably the level is reduced to the level of the matched control or reference group having a low risk of developing CVD. The therapy or administration of the composition according to the invention is preferably delivered to the subject in an amount sufficient to reduce CVD disease incidence or CVD related mortality with at least 10, 20, 30, 50 or 80 % in comparison to a matched control group as defined herein before. Most preferably the composition is administered to reduce progression of and/or stimulate regression of the process of atherosclerosis and the number and size of atherosclerotic lesions with at least 10, 20, 30, 50 or 80% and/or improve cardiac function. Pharmaceutical compositions may optionally comprise excipients known in the art of pharmacy. The medicaments and pharmaceutical compositions according to the invention may be formulated using generally known and pharmaceutically acceptable excipients customary in the art and for instance described in Remington, The Science and Practice of Pharmacy, 21nd Edition, 2005, University of Sciences in Philadelphia. Pharmaceutical compositions and medicaments of the invention may for instance comprise binders such as lactose, cellulose and derivatives thereof, polyvinylpyrrolidone (PVP), humectants, disintegration promoters, lubricants, starch and derivatives thereof, sugar solubilizers or other excipients. The compositions according to the invention may also be comprised in foodstuffs and/or be formulated as a nutraceutical composition, capable of reducing plasma levels of apoE in a subject. Such a nutraceutical composition preferably is in the form of liquid or semi-solid diary composition or in the form of an energy bar or shake, a ready made meal or infusion fluid.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

### Figure legends

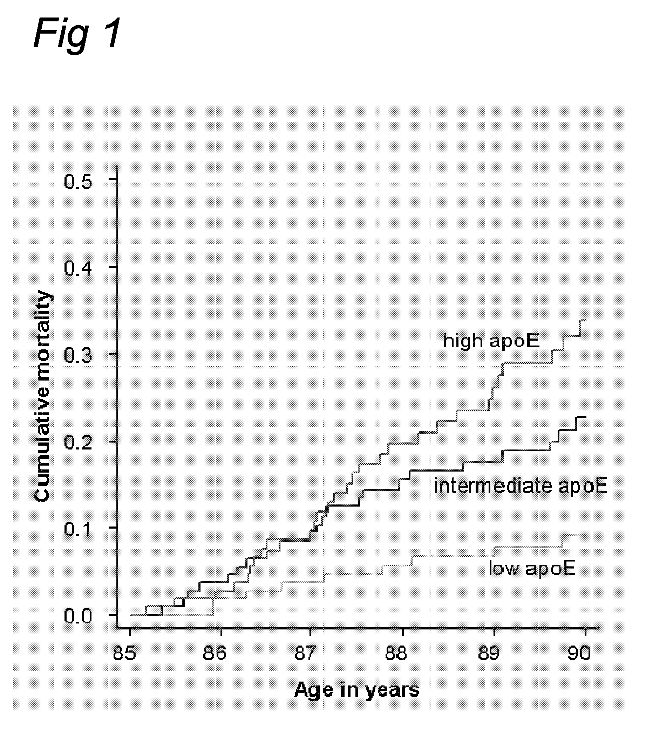

Figure 1. Cumulative mortality from cardiovascular diseases according to tertiles of plasma apoE level in 324 subjects who carried the ε3ε3 genotype.

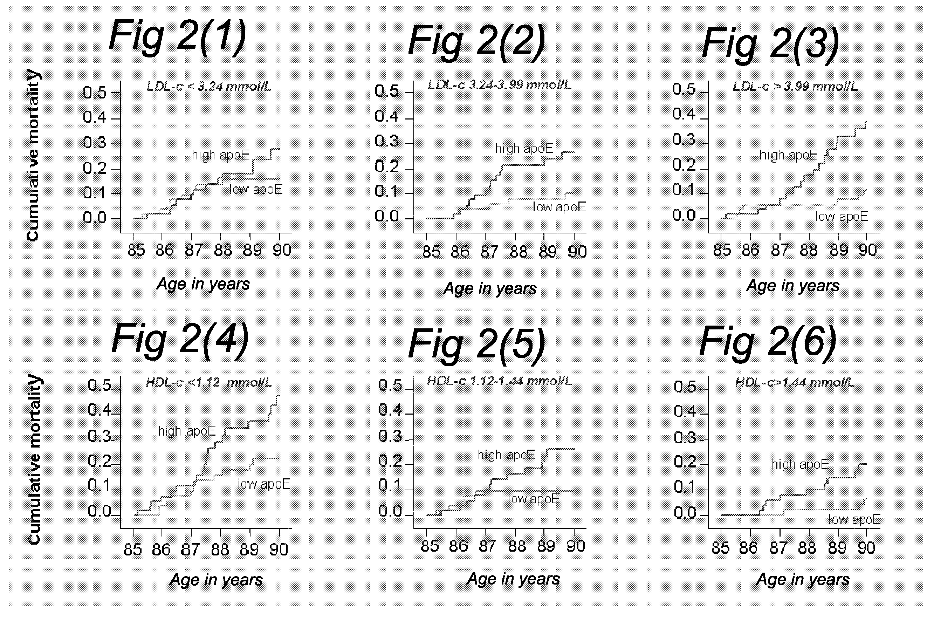

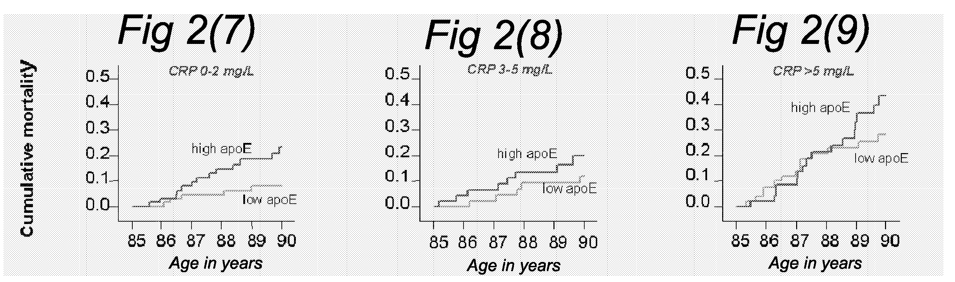

Figure 2. Cumulative mortality from cardiovascular diseases according to high and low plasma apoE level in 324 subjects with the ε3ε3 genotype, stratified for tertiles of LDL-and HDL-cholesterol, and CRP at baseline. Sex adjusted hazard ratios (95% confidence interval) for subjects with a high compared to a low plasma apoE level in various strata using a cox regression; low LDL: 1.66 (0.68-4.09), intermediate LDL 2.71 (0.96-7.61), high LDL 4.25 (1.63-11.0); low HDL: 2.44 (1.15-5.16), intermediate HDL: 3.27 (1.14-9.38), high HDL: 3.69 (0.99-13.7); low CRP: 3.16 (1.12-8.89), intermediate CRP: 2.41 (0.76-7.67), high CRP: 1.56 (0.74-3.29).

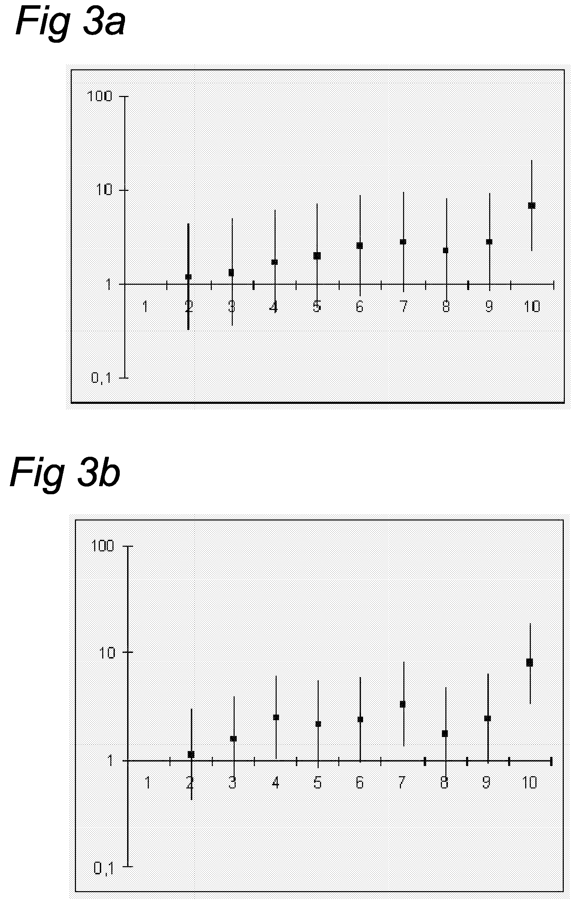

Figure 3. Cardiovascular mortality risk over deciles of plasma apoE, panel A; restricted to homozygote ε3ε3 carriers (n=324), panel B; Whole population in the analysis with *APOE* genotype as covariate in the model. The risk factor of the first decile, i.e. the 10% of the total population having the lowest apoE serum levels, was arbitrarily set as 1 (as reference).
Figure 4. Distribution of apoE plasma levels in ε3ε3 carriers (n=324). In the group of ε3ε3 carriers (n=324), every 1 mg/dl increase in plasma apoE level correlates with an increase in the cardiovascular hazard ratio (sex adjusted) with a factor of 1.12 (95% confidence interval: 1.07-1.18).

### Examples

### Example 1

From 561 eligible subjects, all aged 85 years, measurement of plasma apoE failed in two and *APOE* genotyping in thirteen subjects. The remaining 546 participants were included in the present analysis of which the baseline characteristics are listed in table 1. The *APOE* allele frequencies in our population are: ε2: 0.10, ε3: 0.77, and ε4: 0.13. The genotypes were in Hardy-Weinberg equilibrium and the allele frequencies are in line with those of comparable populations (14).

To eliminate the potentially distorting effect of structural changes in the apoE proteins, the relations between plasma levels of apoE and fractionated cholesterol were estimated in homozygote ε3ε3 carriers only (Table 2). Subjects with high levels of apoE had significantly higher levels of both total- and LDL-cholesterol, and triglycerides (all p<0.001). Plasma levels of HDL-cholesterol were lower, although borderline statistically significant (p=0.051).

Estimates of mortality risk dependent on plasma levels of apoE are listed in Table 3. Again, the sample was restricted to subjects with the ε3ε3 homozygote genotype only. The group was divided into three tertiles of high, medium and low plasma levels of apoE. In this population, subjects with high levels of plasma apoE had a twofold higher all cause mortality risk (hazard ratio, 2.05; 95% confidence interval, 1.39 to 3.02) as compared to subjects with low levels. This was predominantly due to a threefold increase in cardiovascular mortality risk (3.01; 95% confidence interval, 1.60 to 5.66; Figure 1) whereas other causes of death were non-significantly increased. Adjustment for levels of LDL-cholesterol, HDL-cholesterol and triglycerides did not affect the risk estimates (Table 3). Additional adjustment for circulating levels of CRP at baseline only slightly decreased the risk of high plasma apoE levels for cardiovascular mortality (2.42; 95% confidence interval, 1.19 to 4.89; Table 3). Finally, additional adjustment for other classical risk factors had no effect (2.56; 95% confidence interval, 1.23 to 5.31; Table 3).

**Table 1. Baseline characteristics of study participants.**

| | |
|---|---|
| Total number (n) | 546 |
| Sex | |
| Female (number, %) | 365 (67%) |
| Male (number, %) | 181 (33%) |
| Living status | |
| Independent (number, %) | 449 (82%) |
| Institutionalised (number, %) | 97 (18%) |
| Cardiac risk factors | |
| Diabetes mellitus (number, %) | 87 (16%) |
| Hypertension (number, %) | 287 (53%) |
| History of smoking (number, %) | 268 (49%) |
| APOE genotype | |
| ε2ε2 (number, %) | 4 (1%) |
| ε2ε3 (number, %) | 90 (17%) |
| ε2ε4 (number, %) | 13(2%) |
| ε3ε3 (number, %) | 324 (59%) |
| ε3ε4 (number, %) | 100 (18%) |
| ε4ε4 (number, %) | 15 (3%) |
| Plasma apolipoprotein levels, median (IQR) | |
| apoE in mg/dL | 5.02 (3.52-7.20) |
| Plasma CRP level, median (IQR) | |
| C-reactive protein | 4.0 (1.0-8.0) |
| Plasma lipid levels, mean (SD) | |
| Total cholesterolin mmol/L | 5.71 (1.13) |
| LDL cholesterol in mmol/L | 3.68 (0.98) |
| HDL cholesterol in mmol/L | 1.32 (0.40) |
| Triglycerides in mmol/L | 1.57 (0.82) |

**Table 2. Plasma levels of apoE, fractionated cholesterol, triglycerides and CRP according to tertiles of plasma levels of apoE in subjects with the ε3 ε3 genotype.**

| | apoE plasma levels in ε3ε3 subjects | | | |
|---|---|---|---|---|
| | low (n=108) | intermediate (n=108) | high (n=108) | P for trend |
| Apolipoprotein E | 3.06 | 4.87 | 8.71 | nd |
| (median [IQR], mg/dL) | (2.58-3.44) | (4.38-5.38) | (6.85-10.4) | |
| Total cholesterol | 5.43 | 5.60 | 6.08 | <0.001* |
| (mean [SD], mmol/L) | (0.85) | (1.03) | (1.19) | |
| LDL cholesterol | 3.49 | 3.66 | 3.94 | <0.001* |
| (mean [SD], mmol/L) | (0.71) | (0.88) | (1.08) | |
| HDL cholesterol | 1.37 | 1.29 | 1.27 | 0.051* |
| (mean [SD], mmol/L) | (0.36) | (0.37) | (0.41) | |
| Triglycerides | 1.25 | 1.43 | 1.90 | <0.001* |
| (mean [SD], mmol/L) | (0.58) | (0.58) | (0.85) | |
| C-reactive protein | 2.5 | 3.5 | 4.0 | <0.001** |
| (median [IQR], mg/L) | (1.0-4.0) | (1.0-8.0) | (2.0-10.0) | |

| | | | | |
|---|---|---|---|---|
| IQR: interquartile range; nd: not determined. * P for trend calculated using ANOVA ** P for trend calculated using Jonckheere-Terpstra test | | | | |

**Table 3. Mortality risk according to tertiles of plasma levels of apoE in subjects with the ε3ε3 genotype.***

| Cause of death | apoE plasma level | | | P for trend |
|---|---|---|---|---|
| | Low (n=108) | Intermediate (n=108) | High (n=108) | |
| All causes | 1 (ref) | 1.47 (0.98-2.19) | 2.05 (1.39-3.02) | <0.001 |
| Cardiovascular disease | 1 (ref) | 2.12 (1.11-4.06) | 3.01 (1.60-5.66) | 0.001 |
| adjusted for lipids (a) | 1 (ref) | 2.01 (1.04-3.91) | 3.09 (1.55-6.15) | 0.001 |
| adjusted for lipids + CRP (b) | 1 (ref) | 1.72 (0.88-3.35) | 2.42 (1.19-4.89) | 0.014 |
| adjusted for all risk factors (c) | 1 (ref) | 1.78 (0.91-3.48) | 2.62 (1.28-5.39) | 0.008 |
| | | | | |
| Infectiousdisease | 1 (ref) | 1.36 (0.52-3.55) | 1.80 (0.70-4.61) | 0.218 |
| Cancer | 1 (ref) | 2.26 (0.90-5.71) | 2.26 (0.87-5.90) | 0.095 |
| Other causes | 1 (ref) | 0.54 (0.21-1.42) | 1.21 (0.54-2.70) | 0.740 |

| | | | | |
|---|---|---|---|---|
| * All estimates (95% confidence intervals) from cox regression controlled for sex. Models adjusted for other risk factors additionally controlled for; a) plasma levels of LDL cholesterol, HDL cholesterol, triglycerides, b) lipids and circulating levels of CRP, c) lipids, CRP and a history of smoking, diabetes, and hypertension. Ref: reference group. | | | | |

Figure 2 illustrates the risk of cardiovascular mortality associated with high versus low levels of plasma apoE that appears to be independent of the plasma levels of LDL- and HDL-cholesterol, as well as levels of C-reactive protein. Low levels of HDL-cholesterol and high levels of CRP were strongly and significantly associated with increased cardiovascular mortality, whereas LDL-cholesterol did not affect cardiovascular mortality (data not shown).

A more detailed analysis on the group of homozygous ε3ε3 carriers reveals that also a stratification into 10 subgroups (deciles) with increasing apoE serum levels gives a steady increase in a linear fashion. Figure 3 plots the cardiovascular mortality risk over deciles of plasma apoE. Panel A is restricted to homozygote ε3ε3 carriers (n=324), and panel B shows data for the whole population in the analysis with APOE genotype as covariate in the model. The risk factor of the first decile, i.e. the 10% of the total population having the lowest apoE serum levels, was arbitrarily set as 1 (as reference).

The distribution of apoE plasma levels in ε3ε3 carriers (n=324) is shown in figure 4. In the group of ε3ε3 carriers (n=324), every 1 mg/dl increase in plasma apoE level correlates with an increase in the cardiovascular hazard ratio (sex adjusted) with a factor of 1.12 (95% confidence interval: 1.07-1.18).

Subjects who were carriers of the ε2 allele had significantly higher plasma levels of ApoE whereas levels of LDL- and total-cholesterol were significantly lower (Table 4). Inversely, carriers of the ε4 allele had lower levels of plasma ApoE whereas levels of LDL- and total-cholesterol were significantly higher (Table 4).

The risk of mortality from specific causes dependent on the carriership of the various *APOE* alleles is shown in Table 5. In the crude analysis, carriership of the ε2 allele is associated with a non-significant decrease in all cause mortality, although there is a significantly five-fold decreased risk of death from cancer. Carriership of the ε4 allele is associated with a significantly increased all cause mortality risk, that is accounted for by an increased risk of death from 'other causes', a category that includes dementia. To evaluate the risk of the various structural properties of the apoE protein independent of the plasma level, we repeated the analyses of the *APOE* genotype after entering the apoE plasma level as a covariate into the models. From these adjusted models it appears that carriership of the ε2 allele was associated with a significant decreased all cause mortality risk that is explained by a significantly lower risk of death from cardiovascular causes and cancer (Table 5). Carriership of the ε4 allele was associated with significantly increased all cause mortality risk due to a higher risk of death from cardiovascular and 'other' causes (Table 5).

**Table 4. Plasma levels of apoE, fractionated cholesterol, and triglycerides according to APOE genotype.**

| | *APOE* genotype | | |
|---|---|---|---|
| | ε2 carriers (n=94) | ε3 homozygotes (n=324) | ε4 carriers (n=115) |
| Apolipoprotein E | 6.79 | 4.99 | 4.22 |
| (mean [SD]) | (1.71) ‡ | (1.66) | (1.72) † |
| Total cholesterol | 5.27 | 5.70 | 6.12 |
| (mean [SD]) | (1.07) ‡ | (1.07) | (1.21) ‡ |
| LDL cholesterol | 3.16 | 3.70 | 4.10 |
| (mean [SD]) | (0.80) ‡ | (0.92) | (1.07) ‡ |
| HDL cholesterol | 1.35 | 1.31 | 1.29 |
| (mean [SD]) | (0.37) | (0.38) | (0.48) |
| Triglycerides | 1.60 | 1.53 | 1.62 |
| (mean [SD]) | (0.89) | (0.73) | (0.86) |

| | | | |
|---|---|---|---|
| For each comparison, the ε3/ ε3 genotype served as the reference group. Independent samples t-test: † ≤ 0.01; ‡≤ 0.001 Thirteen subject with the ε2 ε4 genotype were discarded from the analysis. | | | |

**Table 5. Mortality risk according to APOE genotype.**

| Cause of death | *APOE* genotype | | |
|---|---|---|---|
| | ε2 carriers n=95 | ε3ε3 n=324 | ε4 carriers n=116 |
| *Crude analysis* | | | |
| All cause | 0.75 (0.53-1.07) | 1 (ref) | 1.44 (1.09-1.91) † |
| Cardiovascular | 0.69 (0.39-1.20) | 1 (ref) | 1.41 (0.93-2.18) |
| Infectious | 1.20 (0.58-2.49) | 1 (ref) | 1.20 (0.59-2.48) |
| Cancer | 0.21 (0.05-0.97)\| | 1 (ref) | 1.15 (0.58-2.79) 2.01 |
| Other cause | 0.82 (0.38-1.79) | 1 (ref) | (1.13-3.55)\| |

| *Adjusted for plasma levels of apoE* | | | |
|---|---|---|---|
| All cause | 0.61 (0.43-0.88) † | 1 (ref) | 1.65 (1.24-2.19) ‡ |
| Cardiovascular | 0.52 (0.30-0.93)\| | 1 (ref) | 1.77 (1.14-2.76)\| |
| Infectious | 1.03 (0.49-2.18) | 1 (ref) | 1.32 (0.63-2.75) |
| Cancer | 0.18 (0.04-0.76)\| | 1 (ref) | 1.25 (0.62-2.52) |
| Other cause | 0.73 (0.33-1.61) | 1 (ref) | 2.14 (1.20-3.82) † |

| | | | |
|---|---|---|---|
| * All estimates (95% confidence intervals) from cox regression controlled for sex. For each comparison, the ε3ε3 genotype served as the reference group. Adjusted models additionally controlled for plasma levels of apoE. Difference from ε3ε3 homozygote subjects: \|≤0.05 ; † ≤ 0.01; ‡≤ 0.001 Thirteen subject with the ε2ε4 genotype were discarded from the analysis. | | | |

### Example 2

To investigate whether the association of plasma apoE with cardiovascular mortality found in ε3ε3 is also present in the other genotypes, we repeated the mortality analysis in other genotypes. We found a similar increase in cardiovascular mortality for subjects in the highest apoE tertile compared to the lowest tertile in ε2 carriers (2.74; 95% confidence interval, 0.49 to 15.3; Table 5) and ε4 carriers (3.72; 95% confidence interval, 1.19 to 11.6; Table 6).

**Table 6. Cardiovascular mortality risk according to tertiles plasma levels of apoE in subgroups of subjects with different APOE genotype.**

| | plasma apoE level | | | P-value |
|---|---|---|---|---|
| | Low | Intermediate | High | |
| ε2 carriers (a) | 1 (ref) | 1.24 (0.23-6.61) | 2.74 (0.49-15.3) | 0.253 |
| ε3ε3 (a) | 1 (ref) | 1.78 (0.91-3.48) | 2.62 (1.28-5.39) | 0.008 |
| ε4 carriers (a) | 1 (ref) | 2.97 (1.05-8.35) | 3.72 (1.19-11.6) | 0.022 |
| All (b) | 1 (ref) | 2.03 (1.24-3.33) | 3.08 (1.79-5.30) | <0.001 |

| | | | | |
|---|---|---|---|---|
| a) Adjusted for sex, lipids, CRP and a history of smoking, diabetes, and hypertension. b) Adjusted for sex, lipids, CRP and a history of smoking, diabetes, and hypertension and *APOE* genotype | | | | |

Concludingly, the main finding of this invention is that subjects with high plasma levels of ApoE are at an increased risk of cardiovascular mortality, independent of their *APOE* genotype and levels of fractionated cholesterol and triglycerides. To assess that the risk of high ApoE plasma levels for cardiovascular mortality was independent of the *APOE* genotype, we analyzed carriers of only *APOE* ε3ε3 genotype separately. Importantly, the correlation between high plasma apoE and CVD mortality found in ε3ε3 carriers was also present in ε2 and ε4 carriers (table 6). To assess that the effect of ApoE plasma levels on cardiovascular mortality was independent of other risk factors, we showed that the risk of a high ApoE plasma level persisted in strata of fractionated cholesterol levels and of CRP, whereas the risk estimate endured when we controlled for all classic risk factors of atherosclerosis by multivariable regression.

In this study population, as well as in other (younger) populations, ApoE levels associate with fractionated levels of total cholesterol, HDL-cholesterol, LDL-cholesterol and triglycerides. At young and middle age, high LDL- and low HDL-cholesterol are strong risk factors of cardiovascular disease. In contrast, in old age only levels of HDL-cholesterol but not the levels of total cholesterol, LDL-cholesterol and triglycerides affect cardiovascular disease risk (2-4). As the association of ApoE levels with HDL-cholesterol is relatively weak, and levels of total cholesterol, LDL-cholesterol and triglycerides do not affect cardiovascular mortality in old age, it is surprising that the plasma level of ApoE is such a strong risk factor of cardiovascular mortality.

The plasma level of apoE is highly dependent on heritable factors (9,10) and we found, as in other (younger) study populations, that *APOE* genotypes associate with the plasma levels of apoE. Interestingly, the *APOE* genotype is only a minor determinant of these levels (15,16) and in accordance the plasma levels of apoE varied markedly in the ε3ε3 carriers. The effect of the plasma levels of apoE masks the effect of the common *APOE* ε2/ε3/ε4 genotypes and may explain why the reported associations of the genotype have been contradicting; the effect of the genotype should be adjusted for plasma apoE levels.

ApoE is a high affinity ligand for hepatic receptors, which are involved in clearance of apoE-rich particles from the circulation such as remnants of chylomicrons and VLDL. (17). High circulating apoE levels are indicative of reduced hepatic clearance of such particles. The increased risk of cardiovascular mortality associated with high apoE levels may therefore at least in part be by caused by the increased retention time of proatherogenic particles in the circulation. Circulating pro-atherogenic particles not taken up by the liver can enter the vessel wall and macrophages thus contributing to foam cell formation. The effects of high apoE may thus be mediated not by the different levels of fractionated cholesterol per se, but by their changed artherogenicity, due to accumulation of specific pro-atherogenic subfractions (such as remnants) or changes in particle sizes. Subtle changes in particle size have been associated with cardiovascular disease (18).

We conclude that plasma apoE levels predict cardiovascular mortality independent of *APOE* genotype, fractionated levels of cholesterol and CRP. These results can readily be exploited for both diagnostic and therapeutic purposes for CVD, aimed at measuring and decreasing plasma levels of apoE.

### Materials and methods

### Participants

Between September 1, 1997, and September 1, 1999, a total of 705 inhabitants of the community of Leiden, the Netherlands, reached the age of 85 years. Among these 85-year-old persons, we initiated a follow-up study to investigate determinants of successful aging. There were no selection criteria on health or demographic characteristics. Fourteen inhabitants died before they could be enrolled. The response rate was 87%; a total of 599 subjects (397 women and 202 men) participated (13). There were no significant differences in various demographic characteristics between the 599 respondents and the source population. Of the 599 participants in the cohort, 38 refused to provide a blood sample, yielding a total number of 561 participants for the present study. The Medical Ethical Committee of the Leiden University Medical Center approved the study, and informed consent was obtained for all subjects.

### APOE genotypes

For genotyping two TaqMan assay (Applied Biosystems) were used. For the single nucleotide polymorphism (SNP) in codon 112 an assay was used that was designed by others (L. Bathum, personal communication); primers were sense 5'-GCTGGGCGCGGACAT-3' and antisense 5'-CACCTCGCCGCGGTACT-3' and probes were 5'-CGGCCGCGCACGTCC-3' labeled with FAM and 5'-AGGCGGCCGCACACGTC-3' labeled with VIC. For the SNP in codon 158 an Assay-On-Demand (Applied Bioosytems) was available with assay ID C____904973_10. The assays were run on a 7900HT (Applied Biosystems) according to manufacturer's specifications, with the following modifications: Eurogentec qPCR core kit was used according standard specifications; half the concentrations of primers and probes were used; the number of PCR cycles was 50. Fluorescence intensities were measured after the PCR and genotypes were indicated by the Sequence Detection Software version 2.0 (Applied Biosystems).

### Plasma parameters

At baseline, subjects were visited twice at their place of residence within one month after the subject's 85th birthday. All blood samples were collected early in the morning, although not fasting. Plasma apoE levels were determined using a human apoE-specific sandwich ELISA. Hereto, an affmity purified polyclonal sheep anti-human apoE antibody was coated overnight onto Maxi sorp immuno plates (Nunc Intermed, Roskilde, Denmark) (dilution 1:10³) at 4°C and incubated with diluted human serum (dilution 1:8,000), overnight at 4°C. Subsequently, affinity purified horse radish peroxidase (HRP)-conjugated polyclonal sheep anti-human apoE antibody (dilution 1:10³) was added, incubated for 2 h at 37°C, and HRP was detected by incubation with tetramethylbenzidine (Organon Teknika, Boxtel, The Netherlands) for 20 min at room temperature. Plasma from C57B1/6 mice spiked with human recombinant apoE (a kind gift from Tikva Vogel, Bio-Technology General, Rehovot, Israel) was used as a standard.

Plasma levels of total cholesterol, HDL cholesterol, triglycerides, and c-reactive protein were analyzed on fully automated computerized analyzers (Hitachi 747 and 911; Hitachi, Ltd, Tokyo, Japan). The level of LDL cholesterol was estimated by the Friedewald equation (LDL cholesterol [mmol/L] = total cholesterol - HDL cholesterol - [triglycerides/2.2]), whereby subjects with a triglycerides concentration higher than 443 mg/dL (5 mmol/L) were excluded (n = 5).

### Causes of death

For the analyses presented in this research, all subjects were followed up for mortality for until April 1st, 2004. The date of death was obtained from the civic registries. Shortly after the civic registries reported the death of a subject, the general practitioner or nursing home physician was interviewed to determine the cause of death by means of a standardized questionnaire. Two senior specialists of internal medicine, unaware of the outcomes of the analyses, reviewed the causes of death and classified each death into primary causes of death according to the *International Classification of Diseases and Related Health Problems, 10th Revision (ICD-10);* cardiovascular mortality (ICD-codes I00-I99) and non-cardiovascular mortality (all other ICD-codes).

### Other cardiovascular risk factors

Subjects were classified as having diabetes when they met at least one of the following criteria: 1) history of type 2 diabetes obtained from the general practitioner or the subject's treating physician; 2) use of sulfonylureas, biguanides, or insulin, based on information obtained from the subject's pharmacist; or 3) nonfasting glucose of 11.1 mmol/l. Subjects were classified as having hypertension when they met at least one of the following criteria: 1) history of hypertension obtained from the general practitioner or the subjects' treating physician; 2) mean systolic blood pressure of 165 mmHg or diastolic blood pressure of 95 mmHg. Of all subjects length and weight was measured at baseline. Body mass index (BMI) was calculated from these measurements. Self reported history of smoking was categorized in three categories: never smoked, did smoke but not anymore, and current smokers.

### Statistical analyses

Levels of lipoproteins were normally distributed. Levels of plasma apoE and CRP were not normally distributed, and are therefore reported as medians and interquartile range (IQR). Differences in these parameters between groups were compared with ANOVA or corresponding non-parametric tests when appropriate. Mortality risks were estimated using Cox Proportional Hazards model and all were adjusted for gender. All calculations were performed using STATA SE 8 and SPSS 12.01.

### References

1. Mahley RW, Ji ZS. Remnant lipoprotein metabolism: key pathways involving cell-surface heparan sulfate proteoglycans and apolipoprotein E. J Lipid Res 1999; 40(1):1-16.
2. Weverling-Rijnsburger AW, Blauw GJ, Lagaay AM, Knook DL, Meinders AE, Westendorp RG. Total cholesterol and risk of mortality in the oldest old. Lancet 1997; 350(9085):1119-1123.
3. Weverling-Rijnsburger AW, Jonkers IJ, van Exel E, Gussekloo J, Westendorp RG. High-density vs low-density lipoprotein cholesterol as the risk factor for coronary artery disease and stroke in old age. Arch Intern Med 2003; 163(13):1549-1554.
4. Shepherd J, Blauw GJ, Murphy MB, Bollen EL, Buckley BM, Cobbe SM et al. Pravastatin in elderly individuals at risk of vascular disease (PROSPER): a randomised controlled trial. Lancet 2002; 360(9346):1623-1630.
5. Weisgraber KH, Rall SC, Jr., Mahley RW. Human E apoprotein heterogeneity. Cysteine-arginine interchanges in the amino acid sequence of the apo-E isoforms. J Biol Chem 1981; 256(17):9077-9083.
6. Schaeffer AJ. Lipoproteins, nutrition, and heart disease. Am J Clin Nutr. 2002;75:191-212.
7. Eichner JE, Dunn ST, Perveen G, Thompson DM, Stewart KE, Stroehla BC. Apolipoprotein E polymorphism and cardiovascular disease: a HuGE review. Am J Epidemiol. 2002;15:487-95.
8. Herz J, Beffert U. Apolipoprotein E receptors: linking brain development and Alzheimer's disease.Nat Rev Neurosci. 2000 Oct;1:51-8.
9. Smit M, de Knijff P, Rosseneu M, Bury J, Klasen E, Frants R, Havekes L. Apolipoprotein E polymorphism in The Netherlands and its effect on plasma lipid and apolipoprotein levels. Hum Genet. 1988;80:287-92.
10. Siest G, Pillot T, Regis-Bailly A, Leininger-Muller B, Steinmetz J, Galteau MM et al. Apolipoprotein E: an important gene and protein to follow in laboratory medicine. Clin Chem 1995; 41(8 Pt 1):1068-1086.
11. Boerwinkle E, Utermann G. Simultaneous effects of the apolipoprotein E polymorphism on apolipoprotein E, apolipoprotein B, and cholesterol metabolism. Am J Hum Genet 1988; 42(1):104-112.
12. Haddy N, De Bacquer D, Chemaly MM, Maurice M, Ehnholm C, Evans A et al. The importance of plasma apolipoprotein E concentration in addition to its common polymorphism on inter-individual variation in lipid levels: results from Apo Europe. Eur J Hum Genet 2002; 10(12):841-850.
13. Bootsma-van der Wiel AB, van Exel E, de Craen AJ, et al. A high response is not essential to prevent selection bias: results from the Leiden 85-plus Study. J Clin Epidemiol 2002;55:1119-25.
14. Haddy N, De Bacquer D, Chemaly MM, Maurice M, Ehnholm C, Evans A, Sans S, Do Carmo Martins M, De Backer G, Siest G, Visvikis S.The importance of plasma apolipoprotein E concentration in addition to its common polymorphism on inter-individual variation in lipid levels: results from Apo Europe. Eur J Hum Genet. 2002 Dec;10(12):841-50.
15. Beekman M, Heijmans BT, Martin NG, Pedersen NL, Whitfield JB, DeFaire U et al. Heritabilities of apolipoprotein and lipid levels in three countries. Twin Res 2002; 5(2):87-97.
16. Neale MC, de Knijff P, Havekes LM, Boomsma DI. ApoE polymorphism accounts for only part of the genetic variation in quantitative ApoE levels. Genet Epidemio1 2000; 18(4):331-340.
17. Davignon J, Cohn JS, Mabile L, Bernier L. Apolipoprotein E and atherosclerosis: insight from animal and human studies. Clin Chim Acta 1999; 286(1-2):115-143.
18. Barzilai N, Atzmon G, Schechter C, Schaefer EJ, Cupples AL, Lipton R et al. Unique lipoprotein phenotype and genotype associated with exceptional longevity. JAMA 2003; 290(15):2030-2040.
19. Wilson PW, Schaefer EJ, Larson MG, Ordovas JM. Apolipoprotein E alleles and risk of coronary disease. A meta-analysis. Arterioscler Thromb Vasc Biol 1996; 16(10):1250-1255.
20. Ridker PM, Cannon CP, Morrow D, Rifai N, Rose LM, McCabe CH, Pfeffer MA, Braunwald E; Pravastatin or Atorvastatin Evaluation and Infection Therapy-Thrombolysis in Myocardial Infarction 22 (PROVE IT-TIMI 22) Investigators. C-reactive protein levels and outcomes after statin therapy. N Engl J Med. 2005 Jan 6;352(1):20-8.
21. Harris HW, Grunfeld C, Feingold KR, Rapp JH. Human very low density lipoproteins and chylomicrons can protect against endotoxin-induced death in mice. J Clin Invest 1990; 86(3):696-702.
22. Rensen PC, Oosten M, Bilt E, Eck M, Kuiper J, Berkel TJ. Human recombinant apolipoprotein E redirects lipopolysaccharide from Kupffer cells to liver parenchymal cells in rats In vivo. J Clin Invest 1997; 99(10):2438-2445.
23. van den Biggelaar AH, de Craen AJ, Gussekloo J, Huizinga TW, Heijmans BT, Frolich M et al. Inflammation underlying cardiovascular mortality is a late consequence of evolutionary programming. FASEB J 2004; 18(9):1022-1024.
24. Garner, B. et al., Modulation of THP-1 Macrophage and Cholesterol Loaded Foam Cell Apolipoprotein E Levels by Glycosphingolipids, Biochem. and Biophys. Res. Comm. 2002 (290) p 1361-1367.
25. Brand, K et al., Interferon gamma inhibits macrophage apolipoprotein E production by post-translational mechanisms, 1993, J. Clin. Invest. 91, p 2031-2039.
26. Deng, J. et al., Lysosomal degradation and sorting of apolipoprotein E in macrophages. 1995, J. Lipid. Res., 36, p 2129-2140.

## Claims

1. Method for determining the risk of a subject for cardiovascular disease, comprising the step of determining the level of Apolipoprotein E (apoE) protein in a bodily fluid.

2. The method according to claim 1, further comprising the step of correlating the level of apoE measured for the individual with the apoE levels of a matched control group with known apoE levels correlated with a corresponding established risk for developing cardiovascular disease.

3. The method according to claim 2 wherein the matched control group is matched with respect to one or more criteria selected from gender, age, *APOE* genotype, smoking, daily calorie intake, body mass index, hypertension, Diabetes Mellitus, CRP, LDL cholesterol, HDL cholesterol and homocysteine plasma levels.

4. The method according to any of claims 1 -3, wherein the bodily fluid is blood, blood serum or blood plasma.

5. The method according to any of the preceding claims wherein the plasma levels are determined using an ApoE specific antibody or a fragment or derivative thereof.

6. The method according to any of the preceding claims wherein the antibody is directly or indirectly labeled.

7. The method according to any of the preceding claims wherein the plasma levels are determined using ELISA, immunoprecipitation, western blot analysis or protein (micro)arrays.

8. A method according to any of the preceding claims for monitoring changes in cardiovascular risk in a subject in response to administration of medicaments or food substances, comprising determining the plasma apoE levels in the subject according to any of claim 1 to 7, at least once before and once after treatment, and optionally at intervals during treatment.

9. The use of a compound selected from the group of compounds capable of reducing circulating levels of apoE, for the manufacture of a medicament for the prevention or the treatment of cardiovascular disease.

10. The use according to claim 9 wherein the compound is selected from the group consisting of TNF-α, INF-γ, Lactosyl-Ceramide (Lac-Cer), Glucosyl-Ceramide (Glc-Cer), phosphonate-phosphate, diphosphonate, aminodiphosphonates, hydroxyphosphonates, phosphonophosphates, HMG-CoA reductase inhibitors (statins comprising mevastatin, lovastatin, compactin, nisvastatin, atorvastatin, pravastatin, simvastatin fluvastatin), NF κB inhibitors and isoprenylation inhibitors.

11. The use of the compound according to claims 9 or 10 for the prevention or the treatment of atherosclerosis.

12. A pharmaceutical composition for the reduction of plasma levels of apoE in a subject comprising as at least one of the compounds selected from the group of compounds capable of reducing ApoE levels, consisting of TNF-α, INF-γ, Lactosyl Ceramide (Lac-Cer), Glucosyl-Ceramide (Glc-Cer), phosphonate-phosphate, diphosphonate, aminodiphosphonates, hydroxyphosphonates and phosphonophosphates, HMG-CoA reductase inhibitors (statins comprising mevastatin, lovastatin, compactin, nisvastatin, atorvastatin, pravastatin, simvastatin, fluvastatin), NF-κB inhibitors, isoprenylation inhibitors, and further comprising at least one pharmaceutical excipient.
